(19) **Europäisches Patentamt**
**European Patent Office**
**Office européen des brevets**

(11) Publication number: **0 448 395 A1**

(12) # EUROPEAN PATENT APPLICATION

(21) Application number: **91302479.0**

(22) Date of filing: **21.03.91**

(51) Int. Cl.⁵: **A61M 5/32**

(30) Priority: **22.03.90 US 497452**

(43) Date of publication of application:
**25.09.91 Bulletin 91/39**

(84) Designated Contracting States:
**DE FR GB IT SE**

(71) Applicant: **CRITIKON, INC.**
**4110 George Road**
**Tampa Florida 33634 (US)**

(72) Inventor: **Chang, Joseph J.**
**7013 W. Linebaugh Avenue**
**Tampa, Florida 33625 (US)**
Inventor: **Hunt, Mary**
**6517 Seafarer Drive**
**Tampa, Florida 33615 (US)**
Inventor: **Young, Susan**
**12945 Pineway Drive**
**Largo, Florida 34643 (US)**

(74) Representative: **Fisher, Adrian John et al**
**CARPMAELS & RANSFORD 43 Bloomsbury**
**Square**
**London WC1A 2RA (GB)**

(54) **Stickless catheter with interconnecting nose.**

(57)     A catheter device is described with a safety needle guard (30) that covers and protects the needle (24) after use of the device. The device includes a semi-tubular needle housing (20) containing a flash chamber (26) with a hollow needle extending from the distal end of the flash chamber. A tubular needle guard (30) concentrically fits and slides within the needle housing. The needle guard has a longitudinal slot (36) through which the mounting base of the flash chamber passes as the guard slides within the housing. The top of the semi-tubular housing is open so that a user may access the top of the tubular needle guard with the finger to urge the needle guard to an extended position from the distal end of the housing and in a surrounding position about the needle. The proximal end or nose of the needle guard (62) detaches of the needle housing and has a luer fitting for an infusion set. This end fits with an interference into the catheter hub assembly.

EP 0 448 395 A1

# STICKLESS CATHETER WITH INTERCONNECTING NOSE

This invention relates to intravascular (I.V.) catheters and, in particular, to I.V. catheter assemblies which cover the needle point after use to prevent accidental injury from used needles. More particularly, this invention relates to the relationship between the needle guard for such a catheter and the catheter hub assembly.

Intravenous catheters for the infusion of fluids into the peripheral veins of a patient are one of the most common devices used in I.V. therapy. I.V. catheters may be produced in two general forms: through-the-needle catheters, in which a catheter is threaded through the needle cannula and into the vein of a patient, and over-the-needle catheters, in which the needle and concentric outer catheter are inserted into the vein and the needle is withdrawn through the emplaced catheter.

A typical over-the-needle I.V. catheter assembly requires the user to remove and then dispose of a contaminated needle after the needle tip and catheter are properly located in a blood vessel of a patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close to where the user is completing work with the catheter, accidental self-inflicted needle injuries are not uncommon. For reasons of the desirability of protecting the user from exposure to blood borne disease such as hepatitis and AIDS, there is an increasing need to protect the user from accidental needle injury.

A catheter design which is directed toward this need is shown in United States Patent number 4,762,516. The catheter shown in this application includes an elongate body which houses a sliding needle guard. In use, the needle with its surrounding catheter tube is inserted through the skin of a patient until the tip of the needle is located in a blood vessel, a position detected by a small flow of blood through the needle and into the flash chamber of the catheter. The user then advances a tab on the top of the needle guard to simultaneously thread the catheter tube into the blood vessel and begin the retraction of the needle from the catheter tube. As the needle is withdrawn from the emplaced catheter, the advance of the tab slides the needle guard out of the housing and along the needle, until the distal end of the guard covers the needle tip and the proximal end of the guard locks in the elongate body. The needle and guard may then be set aside with the needle tip fully protected.

It is also desirable to provide such a catheter in a smaller, smoothly operating configuration which can be readily manipulated by small hands. In accordance with U.S. Serial Number 335,472 (see EP-A-0392765), assigned to a common assignee as this invention, a catheter assembly with needle guard is provided with a semi-tubular needle housing that is open on the upper surface. Located within the housing is a flash chamber with a needle extending from the distal end of the chamber and beyond the distal end of the housing. A tubular needle guard is located for distal movement within the semi-tubular needle housing, and has a distal opening through which the needle extends. The bottom of the needle guard is slotted to fit around the base of the flash chamber. At the rear of the needle guard slot is a portion of a locking mechanism which will engage with and lock in the needle housing when the needle guard is extended to cover the needle.

While these previously mentioned disclosures effectively operate to cover the needle after removal from the catheter hub assembly, they are somewhat lacking in their ability to prevent seepage of blood while the needle is inserted through the catheter or cannula and into the patient. Normally during insertion, blood flows through the cannula into a flash chamber in the needle assembly. Naturally, blood will flow into the flash chamber until the chamber is full. A porous barrier permits breathing of the flash chamber, and yet prevents blood from leaking through the back of the flash chamber.

In previous designs it would be likely for the blood to flow out of the needle nose and into the void distal area of the catheter hub. In fact, some of the needle/hub assembly configurations increase this likelihood in that the catheter hub and needle nose had a clearance designed between them. This clearance was created for two reasons. First, it was desirable for the needle nose base to be fully seated within the distal end of the hub to assure proper placement of the needle and catheter within the patient.

Second, it was desirable to create a clearance so that during withdrawal of the needle guard from the catheter, there would be no increased force needed to disconnect the needle from the catheter. This made it possible for blood to leak out of the catheter hub or flow through the needle nose to leak within the catheter.

If the catheter contained a gasket at the tip of the needle nose, blood was prevented from being pulled within the needle guard and therefore could leak from only one location, the proximal end of the needle hub. Although such leakage is a rather common phenomenon for an intra I.V. catheter, blood leakage is not a desirable situation, since this may cause inadvertent blood contact, and also make locking and withdrawal of a needle guard more troublesome.

## Summary of the Invention

It is an object of the invention to provide a seal between the catheter hub and needle nose of a catheter assembly.

It is yet another object of the invention to provide a catheter assembly which prevents blood leakage during insertion of the needle into the patient.

It is yet another object of the invention to provide a catheter hub assembly and needle nose where the force to disconnect the needle is not increased even though there is a localized seal between needle nose and catheter hub.

It is yet another object of the invention to provide a catheter in which blood accumulates only in the flash chamber of the catheter assembly until disconnection of the needle from the catheter assembly.

Finally, it is another object of the invention to provide a catheter assembly where the needle nose is formed from a material possessing low creep properties so that an interference is maintained between the catheter hub assembly and the needle nose.

These and other objects of the invention are accomplished in a catheter assembly wherein the I.V. catheter device has standard catheter with luer connecting hub, a slidable needle housing and an extendable needle guard. The nose tip of the needle guard is designed to create a positive interference with the catheter hub assembly, so that the hub/catheter and the needle guard behave as one unit. This interference fit causes a sealing effect between the catheter hub and needle nose assembly. It further allows the needle nose to be fully seated within the catheter hub to maintain accurate control of the distance between the catheter tip and the beveled portion of the needle.

In addition, the current configuration of the needle guard allows the needle guard to remain attached to the hub until withdrawal of the needle within the guard. This prevents rolling or falling of the needle guard during insertion of the needle and catheter assembly or taping of the catheter to the patient. In producing the interference fit between the needle nose and catheter hub assembly, it is desirable to optimize the configuration of the needle nose so that while sealing is effected, the force required to disconnect the guard from the catheter hub remains constant.

In a preferred embodiment of the present invention the needle guard also includes a separate nose piece which enables the mounting of a catheter hub over the needle guard nose. The use of a separate tip also facilitates automated assembly without damage to the sharp pointed needle. When the needle guard is extended, the distal end of the tip piece extends beyond the point of the needle. The needle housing of the preferred embodiment also includes an integral, contoured finger grip located on each side of the needle housing. The catheter assembly is further provided with a protective sheath to protect the catheter and needle prior to use.

The invention will be better understood from the following drawings and Detailed Description of the Invention.

## Detailed Description of the Drawings

FIGURE 1 is perspective view of a catheter assembly constructed in accordance with the principles of the present invention;

FIGURE 2 is a cross-sectional view of the catheter assembly taken along lines 2-2 of Figure 1; and

FIGURE 3 is a cross-sectional view of a needle guard nose suitable of this invention for use with the catheter assembly of Figures 1 and 2.

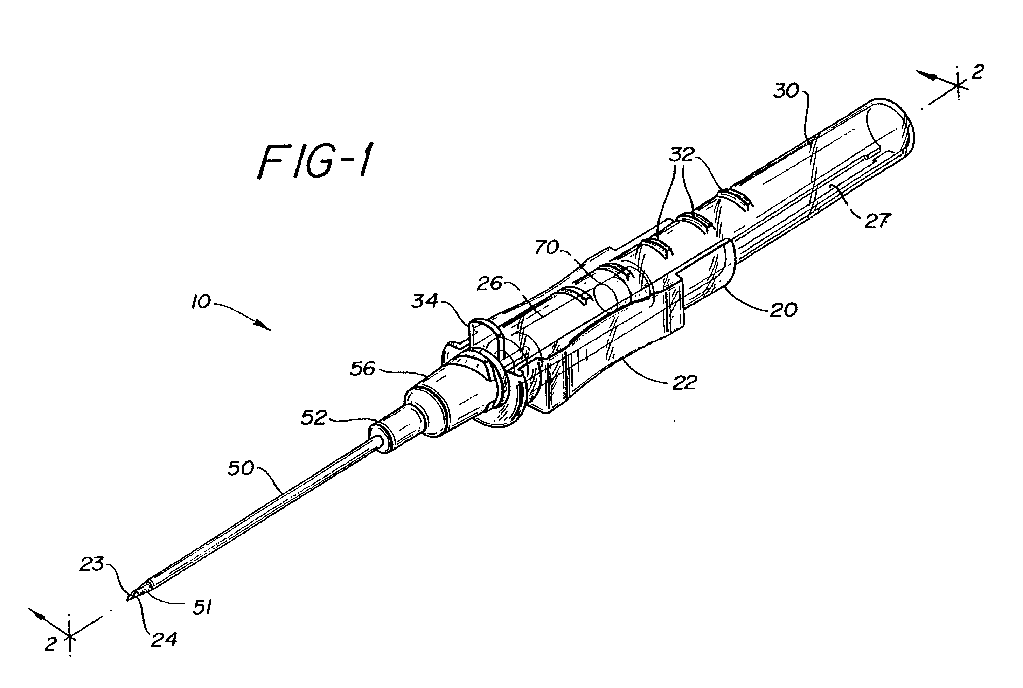

Referring first to FIGURE 1, a catheter assembly 10 constructed in accordance with the principles of the present invention is shown. The assembly 10 includes a needle housing 20 which is semi-tubular in shape and open at the top. Molded on the sides of the needle housing 20 are opposing contoured finger grips 22, one of which is visible in FIGURE 1. Located inside the semi-tubular needle housing and extending proximally therefrom is a tubular needle guard 30. On the upper surface of the needle guard are a number of small projections 32 which provide surfaces against which a user may press to fully extend the needle guard. These projections permit a user to extend the needle guard with the index or other finger while holding the catheter assembly with one hand. Extending distally from the needle housing 20 is a protective sheath (not shown) which covers the distally extending needle and catheter.

The catheter 50 and its catheter hub 52 are mounted on the distal end of the needle guard 30. The point of the needle 24 is seen to extend from the distal tip of the catheter 50. A push-off tab 34 is seen projecting upward from the needle guard proximal the catheter hub 52. Located on the distal end of the needle guard is a needle guard tip 60, through which the needle 24 extends. After the needle guard 30 has been extended to cover the needle 24, the needle guard is locked in its extended position inside the needle housing, and the point of the needle is located inside of the needle guard nose or tip 60.

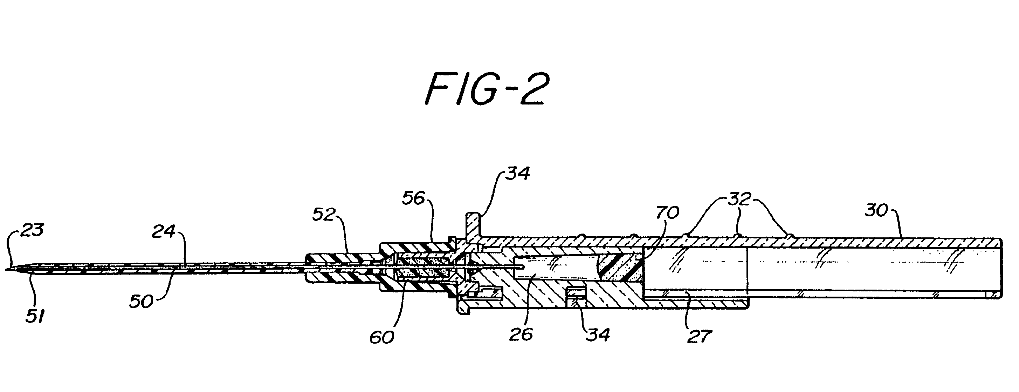

FIGURE 2 is a cross-sectional view of the catheter assembly of FIGURE 1. The catheter 50 is seen to extend from the distal end 54 of the catheter hub 52 and is concentric therewith. The catheter may be attached to its hub by any means known in the art, including adhesively or mechanically by means of a metal eyelet. The larger diameter proximal portion 56 of the catheter hub 52 is flanged at its proximal end for connection to an infusion set, and the inner diameter of the proximal portion of the hub is sized to fit over the distal portion of the needle guard tip 60.

The needle 24 is attached to the distal end of the flash chamber 26 of the needle housing with the proximal end of the needle terminating within the chamber. The needle 24 is affixed in place by adhesive or gasketing mechanisms. The needle extends through the needle guard nose 60, the needle hub 52, and the catheter 50, with the point of the needle extending from the distal end of the catheter. The rear of the flash chamber 26 is plugged by a microporous plug 70. The needle guard is seen to extend proximal the rear of the needle housing with the needle guard nose 60 affixed to the distal end of the needle guard at the location of the push-off tab 34. The tubular needle guard surrounds the flash chamber 26, with the base 27 of the flash chamber being located in a longitudinal slot 36 at the bottom of the needle guard. As the needle guard slides in the distal direction to cover the needle it is maintained concentric with the needle housing by the concentric tubular construction of the needle housing and needle guard and by the tracking of the base 27 of the flash chamber in the needle guard slot 36.

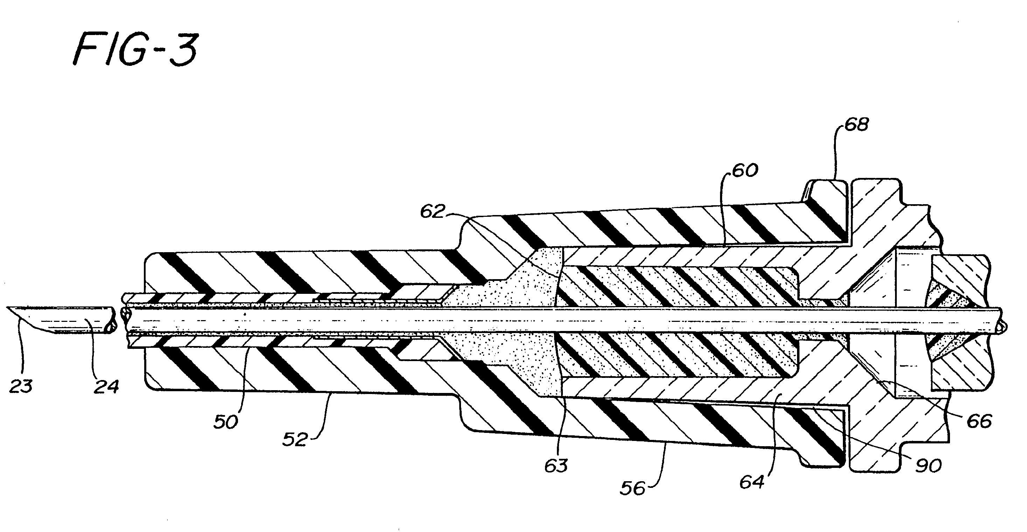

A needle guard nose 60 suitable for use with the needle guard of FIGURE 1-2 is shown in cross-section in FIGURE 3. The proximal end 62 of the nose 60 is sized to fit in the distal opening 90 of the needle guard 30. The proximal end of the tip is inserted into the needle guard until the shoulder 63 of the tip contacts the side walls of the guard. The central section 64 of the nose 60 has walls on both its internal and external surfaces which maintain their proper sizing to be emplaced within the catheter hub. The distal end 66 of the tip 60 is rounded and open for passage of the needle through the tip. There is a luer fitting 68 on catheter hub 52 for attachment of an infusion set.

The proximal end 62 of the nose 60 is designed to create a positive interference with the catheter hub 52 so that a sealing ring effect is achieved. Blood does not flow through the sealing section until the clinician is ready for disconnecting and removal of the locked needle guard device. The proximal end 62 outside diameter is at least 0.0005" and no more than 0.0006" larger than the inside diameter of the catheter hub 52 at a depth equivalent to the length of the nose 60 so that (a) a sealing ring effect is created, (b) the catheter hub 52 can be fully seated on the base of the nose 60 to allow for accurate control of the catheter tip trim assuracy (distance between the catheter tip 51 and the needle bevel 23, (c) the needle guard 30 would maintain attached to the hub 52 until withdrawal and removal of the needle guard 30. This prevents rolling or falling of the needle guard 30 during insertion or taping. Also, the force required to disconnect the guard 30 from the hub 52 is optimized so that the user does not even notice the interference fit at the connection. The nose 60 is formed from a low creep material in order to maintain this interference fit at all times, from manufacturing to final use of the catheter assembly 10.

The catheter assembly of FIGURES 1, 2 and 3 may be used in the conventional manner by inserting the concentric catheter and needle through the skin of a patient and into a blood vessel. When the point of the needle 24 is properly located in the vessel, a small amount of blood will flow through the needle and into the flash chamber 26. Since the needle housing and guard are made of transparent or translucent polymeric materials, the flow of blood will be readily apparent in the flash chamber. Because the needle guard and catheter hub are configured to be maintained in an interference fit, blood enters only the flash chamber 26, and not the catheter hub 52 assembly by seepage from needle guard nose 60. The needle is then retracted from the vessel and the catheter 50 threaded into the vessel by grasping the finger grips 22 of the housing with the thumb and fingers and pushing the push-off tab 34 in the distal direction with one finger. This motion will push the catheter hub 52 off of the needle guard tip 60 to advance the catheter. As the needle guard begins to extend out from the distal end of the needle housing such that the push-off tab 34 is beyond the reach of the finger of the user, the user may engage the projections 32 with the finger to continue the distal motion of the needle guard.

Finally this motion will result in proper threading of the catheter into the vessel and the complete withdrawal of the needle from the patient's body. The needle guard 30 is then advanced to its fullest extension and lock in its protective position. The needle, housing and guard may then be set aside without concern for inadvertent injury to the user or others, and an infusion set is attached to luer fitting 68 in order to begin infusion to the patient.

The invention may be better understood from the attached claims and their equivalents.

## Claims

1. A catheter assembly comprising:
   a catheter hub;
   a needle housing having a distal end;
   a hollow needle extending from the distal end of said needle housing; and
   a needle guard slideably located within said needle housing and including at its distal end means for engaging said catheter hub in an interference fit, said distal end having an aperture for passage of said hollow needle therethrough.

2. The catheter assembly of Claim 1, wherein said needle housing further comprises a flash chamber located within said housing, wherein said hollow needle extends from said flash chamber and the passageway of said hollow needle is in fluid communication with the interior of said flash chamber.

3. The catheter assembly of Claim 2, wherein said flash chamber is located within said slideably mounted needle guard.

4. The catheter assembly of Claim 1, wherein said needle housing further includes two lateral sides, wherein each lateral side includes a contoured finger grip.

5. The catheter assembly of Claim 1, wherein said needle guard further includes an upward extending push tab located in the proximity of the distal end of said needle guard.

6. The catheter assembly of Claim 1 wherein said catheter hub and needle guard are tubular, and said needle guard outer diameter is oversized to fit with said catheter hub inner diameter.

7. The catheter assembly of Claim 6 wherein said interference fit is about 0.005".

8. The catheter assembly of Claim 6 wherein said needle guard has a luer connection for attachment of an infusion set after removal of said needle and said needle housing.

9. The catheter assembly of Claim 9, wherein said needle guard comprises a longitudinal tubular section having a relatively large inner diameter and a distally mounted needle guard tip having a relatively small distal opening for passage about said hollow needle.

10. The catheter assembly of Claim 9, further including a catheter attached to said catheter hub and allowing passage of said needle when said needle guard and catheter hub are placed in said interference fit.

11. A catheter assembly comprising:
   a needle housing having a hollow needle extending from the distal end thereof;
   a needle guard slideably located within said needle housing and including an aperture at its distal end for passage of said hollow needle therethrough;
   a catheter and catheter hub assembly suitable for mounting on the distal end of said needle guard; and
   means for maintaining said needle guard in said catheter hub.

12. The assembly of Claim 11 wherein said maintaining means holds said catheter hub in an interference fit around said needle guard.

13. The assembly of Claim 12 wherein said needle guard has a luer attachment at its distal end, said luer attachment accepting to either an infusion set or said needle housing.

14. The assembly of Claim 12 wherein said interference fit is about 0.005".

15. The assembly of Claim 12 wherein said needle guard is made for a low creep material.

16. The assembly of Claim 12 wherein said catheter, catheter hub, needle, needle guard and needle housing are all hollow and tubular.

FIG-1

EP 0 448 395 A1

**FIG-2**

FIG-3

EP 0 448 395 A1

| | European Patent Office | EUROPEAN SEARCH REPORT | Application Number |
|---|---|---|---|
| | | | EP 91 30 2479 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-343 803 (LUTHER ET AL.)<br><br>* column 3, line 22 - column 6, line 10; figures 1-6 * | 1-10,<br>11-16 | A61M5/32 |
| X | EP-A-322 129 (ICU MEDICAL)<br><br>* column 4, line 26 - column 6, line 50; figures * | 1-3,<br>5-10,<br>11-16 | |
| X | WO-A-8 900 865 (SAGSTETTER ET AL.)<br><br>* page 6, line 1 - page 8, line 30; figures * | 1-3,<br>5-10,<br>11-16 | |
| | | | **TECHNICAL FIELDS SEARCHED (Int. Cl.5 )**<br><br>A61M |

The present search report has been drawn up for all claims

| Place of search | Date of completion of the search | Examiner |
|---|---|---|
| THE HAGUE | 05 JUNE 1991 | MIR Y GUILLEN V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document